# EUROPEAN PATENT APPLICATION

(11) **EP 4 039 166 A1**
(43) Date of publication of application: **10.08.2022**
(21) Application number: 22154089.1
(22) Date of filing: 28.01.2022
(51) Int. Cl.: A61B 1/00, A61B 1/06, A61B 1/07, A61B 17/34, A61B 17/00

(54) **ENDOSCOPE ASSEMBLY HAVING A SURGICAL INSTRUMENT AND ENDOSCOPE SYSTEM HAVING THE SAME**

(30) Priority: 03.02.2021 TW 110201347 U; 30.09.2021 TW 110136442
(71) Applicant: Kao, Hong So, Taipei City 106001 (TW); Chang, Chin Piao, Zaoqiao Township 361027 (TW)
(72) Inventor: Kao, Hong So, Taipei City 106001 (TW); Chang, Chin Piao, Zaoqiao Township 361027 (TW)
(74) Representative: Regimbeau

(57) **Abstract**

An endoscope assembly (1) includes an endoscope device (20) and a surgical instrument (10). The endoscope device (20) includes a base module (40), an injection needle (24) connected to the base module (40), and an image sensing unit (211) . The image sensing unit (211) includes a tube portion (213) extending from the base module (40) into the injection needle (24), and an image sensor (211) adjacent to a bevel surface (242) of the injection needle (24). The surgical instrument (10) includes a connecting rod (12) extending into the injection needle (24), and a forceps device (11) having two pincers (111), a hinge set (112) that is connected between the pincers (111), and a push rod (113) that extends from the hinge set (112) away from the connecting rod (12). Conversion of the forceps device (11) drives operation of the hinge set (112) to move the push rod (113) away from the connecting rod (12).

## Description

The disclosure relates to an endoscope, and more particularly to an endoscope assembly and an endoscope system using the endoscope assembly.

Traditional open surgery requires a large incision to be made on a patient so that the surgical area may be accessed. However, advancements in medical technology have led to the development of minimally invasive surgery that is performed with endoscopes and surgical instruments such as forceps, and that only require relatively small incisions to be made on the patient. Minimally invasive surgical techniques have become widely adopted in a variety of surgical procedures. Examples of minimally invasive surgery include keyhole surgeries such as, arthroscopy, laparoscopy, thoracoscopy, and endoscopy.

A conventional endoscope used in key-hole surgery has a flexible tube and a camera mounted to the end of the tube. It is employed as a diagnostic tool by inserting the endoscope into a patient's body cavity, illuminating an area around the camera so that the camera may capture video and images of the surgical area, sending the captured images and video to an electronic device or diagnostic tool, having a doctor make a diagnosis based on the captured information, and finally performing a surgical operation on the surgical area identified in the diagnosis. The conventional endoscope has a variety of different lens types that are adapted for different medical procedures. Additionally, since surgical instruments are quite specialized and a variety of different functions may need to be performed during the operation, which may include grasping, retracting, incision, resection, excision, etc., an operating surgeon will select the surgical instruments most suitable for the particular surgical operation. During a key-hole surgery operation such as laparoscopy, an incision needs to be made on the patient for the endoscope tube to extend into the patient and find the targeted surgical area, and a trocar is then inserted in the patient making another incision that is puncture-like, for the insertion of a cannula, so that a variety of surgical instruments (such as forceps or scalpels) or syringes may be inserted through the cannula to access the surgical area and perform the necessary treatment on the targeted surgical area.

Keyhole surgery is therefore quite a complex operation involving many procedures; many difficulties may arise during the surgery that may extend the duration of the operation, and this extension is associated with an increased risk of postoperative complications. Additionally, the use of the trocar during the operation may require the patient to be sutured, which may lengthen the patient's post-operation recovery period. Furthermore, the conventional surgical instruments currently used in keyhole surgery are designed for the functions of grasping, retracting, incision, resection, excision, etc., but does not functions such as drug delivery or the delivery of a medical device.

Therefore, an object of the disclosure is to provide an endoscope assembly that can alleviate at least one of the drawbacks of the prior art.

According to one aspect of the disclosure, the endoscope assembly includes an endoscope device, and a surgical instrument. The endoscope device includes a base module, an injection needle, and an image sensing unit. The base module includes an illumination input end. The injection needle is tubular, is connected to the base module, and has a bevel surface formed on a distal end thereof. The image sensing unit includes a tube portion extending from the illumination input end of the base module into the injection needle, and an image sensor connected to a distal end of the tube portion and being adjacent to the bevel surface of the injection needle. The surgical instrument includes a handle, a connecting rod, and a forceps device. The connecting rod has an end connected to the handle, and extends into the injection needle. The forceps device is connected to a distal end of the connecting rod opposite to the handle. The forceps device is convertible between an enclosed state where end portions of the two pincers are locked together and closed, and an open state where the end portions of the two pincers are separated from each other. Conversion of the forceps device toward the open state drives the hinge set to operate to thereby move the push rod away from the connecting rod.

Another object of the disclosure is to provide an endoscope system having the abovementioned endoscope assembly.

According to another aspect of the disclosure, the endoscope system includes the abovementioned endoscope assembly and an output unit. The base module of the endoscope assembly further has an output end. The output unit is connected to the output end of the base module of the endoscope assembly. The output unit has a bus, a processor, and a display device. The bus is connected to the image sensing unit via a data connection. The processor is connected to the bus via a data connection. The display device is electrically connected to the processor. The bus is disposed for transmitting an image signal captured by the image sensor. The processor is disposed for receiving the image signal transmitted by the bus and for processing the image signal. The display device is disposed for displaying the image signal processed by the processor.

Other features and advantages of the disclosure will become apparent in the following detailed description of the embodiments with reference to the accompanying drawings, of which:
Figure 1 is a schematic view illustrating an endoscope assembly of an embodiment of an endoscope system according to the present disclosure;
Figure 2 is an enlarged fragmentary perspective and schematic view showing a forceps device of the endoscope assembly of the embodiment in an enclosed state;
Figure 3 is an enlarged fragmentary perspective view showing a forceps device of the endoscope assembly of the embodiment in an open state;
Figure 4A is an enlarged fragmentary sectional view of the endoscope device of the endoscope assembly of the embodiment;
Figure 4B is a view similar to Figure 4A, but showing a variation of the endoscope device of the endoscope assembly of the embodiment.
Figure 5 is a fragmentary enlarged frontal view of the endoscope device;
Figure 6 is a view similar to Figure 5, but showing a variation of an image sensor of the endoscope device.
Figure 7 is a side view illustrating a surgical instrument of the endoscope assembly of the embodiment;
Figure 8 is an enlarged fragmentary schematic side view of the forceps device;
Figure 9 is a view similar to Figure 8, showing the forceps device in an open state;
Figure 10 is a view similar to Figure 8, showing the forceps device in an enclosed state;
Figures 11A to 11D show variations of the forceps device;
Figure 12 is a view similar to Figure 1, showing the endoscope assembly in an alternative arrangement to Figure 1;
Figure 13 is a schematic block diagram illustrating the image capture and display process of the embodiment; and
Figures 14A to 14C show variations of the configuration of a light source conductor.

Before the disclosure is described in greater detail, it should be noted that where considered appropriate, reference numerals or terminal portions of reference numerals have been repeated among the figures to indicate corresponding or analogous elements, which may optionally have similar characteristics.

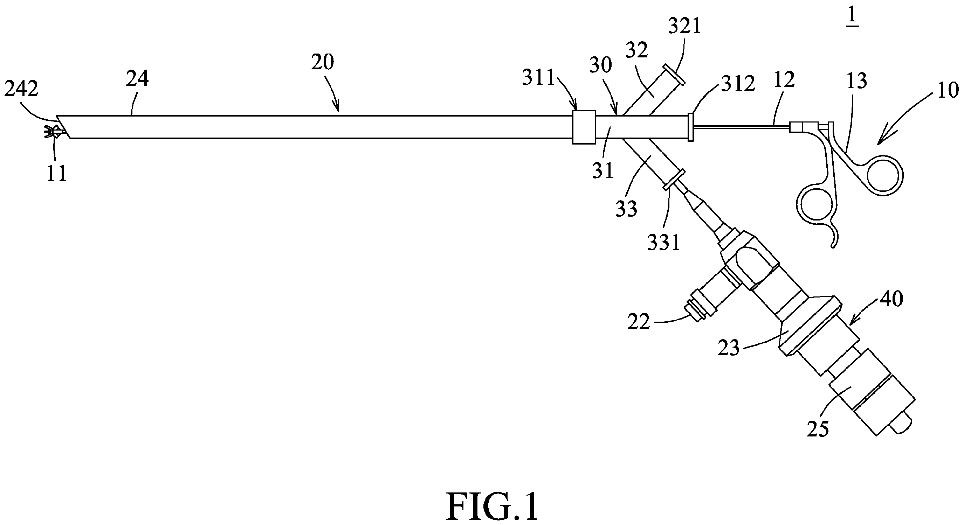

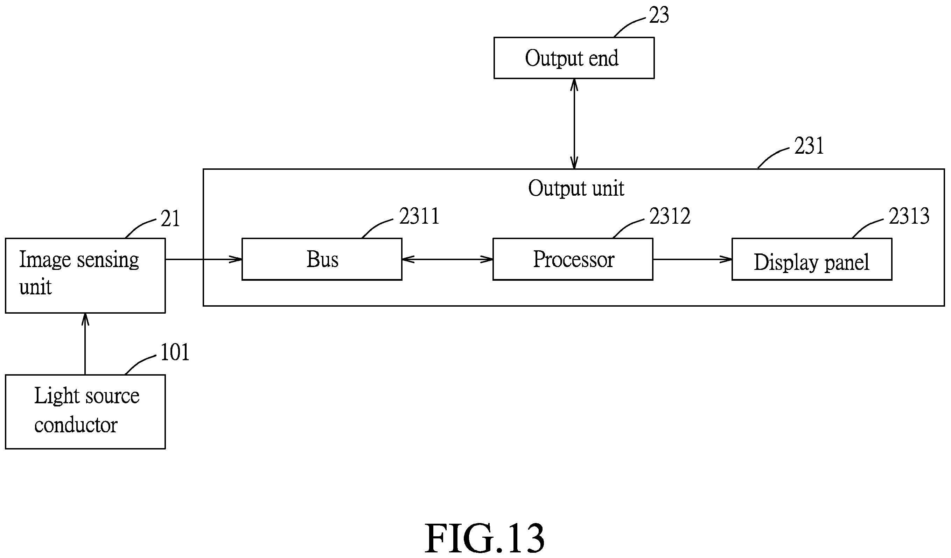

Referring to Figures 1 to 3, and Figure 13, an embodiment of the endoscope system according to the present disclosure includes an endoscope assembly 1 and an output unit 231 (see Figure 13). The endoscope assembly 1 includes an endoscope device 20 and a surgical instrument 10.

Referring to Figures 1 to 3, the endoscope device 20 includes an adapter 30, a base module 40 removably connected to the adaptor 30, an injection needle 24 connected to an end of the adapter 30 that is distal to the base module 40, an image sensing unit 21 extended into the injection needle 24, and an optical component 215 mounted in the injection needle 24. The surgical instrument 10 includes a handle 13, a connecting rod 12 and a forceps device 11.

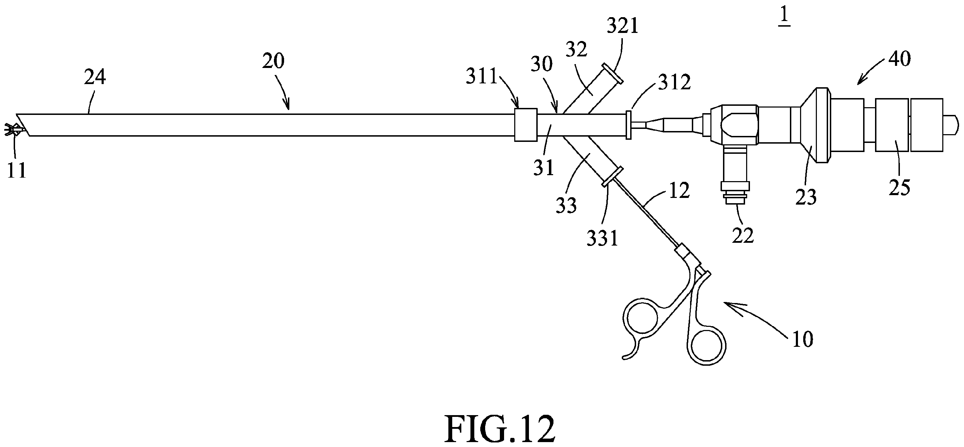

Referring to Figures 1 and 12, the base module 40 includes an illumination input end 22, an output end 23, and a relay mechanism 25 connected to the output end 23. The output unit 231 is connected to the output end 23 of the base module 40. In some embodiments, the endoscope assembly 1 uses fiber optic illumination, in these cases, the illumination input end 22 may be provided with a light source, and the relay mechanism 25 is configured to be an optical fiber light concentrator. However, in other embodiments, the endoscope assembly 1 may be illuminated via other forms of lighting, in these cases, the relay mechanism 25 can be configured as an LED light power source, or a multimedia interface. Additionally, the output unit 231 can be connected to the output end 23 of the base module 40 via a wireless connection. In this case, the relay mechanism 25 can have a wireless communication module attached; in cases where wireless connection is not required a traditional wired communication module may be substituted. However, the relay mechanism 25 can be omitted in other embodiments. For example, the output unit 231 can be a head-mounted display (HMD), so that an operating surgeon may be provided with a live feed video captured by the image sensing unit 21. Additionally, the illumination input end 22 may be the sole component included in the base module 40, thereby reducing the size of the base module 40; the reduced size and weight of the base module 40 will allow the operating surgeon to dexterously operate the endoscope assembly 1. In this embodiment, the light provided through the illumination input end 22 may be adjusted to a particular wavelength that is most suited to the requirements of the surgical operation being performed.

Referring to Figures 1 and 12, the adaptor 30 includes a first branch tube 31, a second branch tube 32, and a third branch tube 33. The first branch tube 31 has two opposite ends respectively formed with a first instrument port 312 and a connection port 311. The second branch tube 32 is connected to the first branch tube 31 and has an injection port 321. The third branch tube 33 is connected to the first branch tube 31 and has a second instrument port 331. The connection port 31, the first instrument port 312, the injection port 321 and the second instrument port 331 are in fluid communication with each other. The connection port 311 is connected to the injection needle 24, and the injection port 321 is adapted for inlet of a pharmaceutical therethrough into the injection needle 24. The base module 40 can be removably connected to one of the first instrument port 312 and the second instrument port 331, and the surgical instrument 10 can be extended into the injection needle 24 via the other one of the first instrument port 312 and the second instrument port 331, so that the handle 13 of the surgical instrument 10 is space apart from the base module 40, thereby allowing the operating surgeon to have more space and not be obstructed by the base module 40 while manipulating the surgical instrument 10. Additionally, the removable connection of the base module 40 with the adaptor 30 allows an operator of the endoscope assembly 1 to disconnect and remove the base module 40 when it is not needed. In this embodiment, the first branch tube 31 is straight, the third branch tube 33 obliquely extends from the first branch tube 31 away from the connection port 311, and the third branch tube 33 and the first branch tube 31 form an acute angle therebetween. Referring to Figure 1, in this embodiment, the surgical instrument 10 is extended through the first branch tube 31 into the injection needle 24 via the first instrument port 312; this avoids bending the surgical instrument 10, allows for a straight extension of the surgical instrument 10 into the injection needle 24, and makes the control of the surgical instrument 10 intuitive and easy to manipulate for the operating surgeon. The base module 40 is connected to the second instrument port 331, the light source is provided from the illumination input end 22, the light passes through the third branch tube 33, and toward the image sensing unit 21. However, referring to Figure 12, since the connecting rod 12 of the surgical instrument 10 is flexible in this embodiment, the surgical instrument 10 can alternatively extend obliquely through the third branch tube 33 into the injection needle 24 via the second instrument port 331. The base module 40, on the other hand, is removably connected to the first instrument port 312. In this arrangement, the surgeon will have plenty of space to operate the surgical instrument 10.

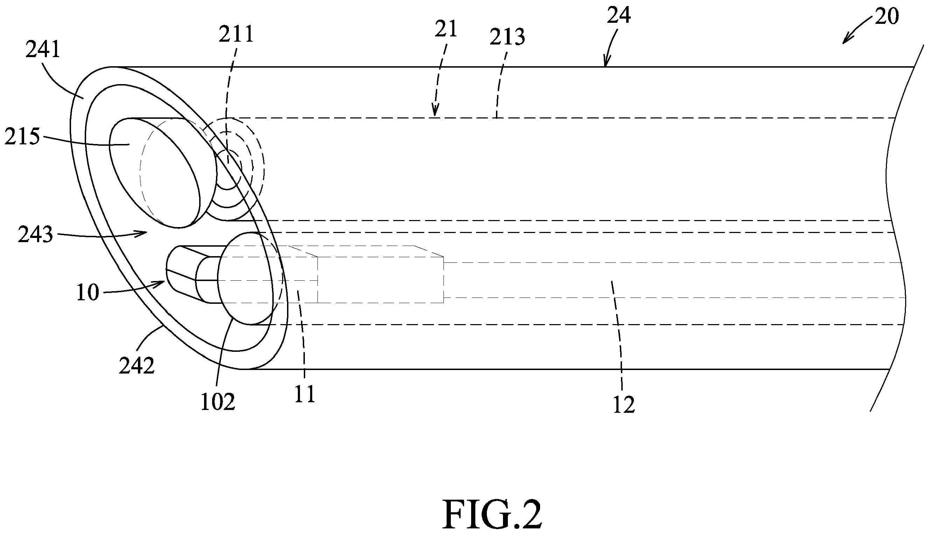

Referring to Figures 1 and 2, the injection needle 24 is tubular, is connected to the connection port 311 of the base module 30, and has a bevel surface 242 that is formed on a distal end thereof, and an end hole 243 formed in the bevel surface 242. The end hole 243 is adapted to outlet the pharmaceutical that was inlet from the injection port 321. In this embodiment, the image sensor 211 has an end surface that is perpendicular to an axis of the injection needle 24, and the bevel surface 242 of the injection needle 24 is inclined relative to the end surface of the image sensor 211. The inclined bevel surface 242 forms a sharp tip 241 that is adapted to puncture a patient's skin. This allows the endoscope device 20 to directly extend into the patient with the puncturing of the injection needle 24, which is much more convenient and quicker than the conventional procedure, which required making an incision on the patient with a trocar and installing a cannula in the incision for the endoscope to extend. The puncture wound of the injection needle 24 is also smaller which aids postoperative recovery. In this embodiment, the outer diameter of the injection needle 24 is in a range from 1.0 millimeters to 2.5 millimeters; the puncture wound diameter on the patient will be no larger than 2.5 millimeters, which generally does not require sutures for closing the wound, and thereby reduces the postoperative recovery period. Additionally, in this embodiment, the injection needle 24 is removably connected the base module 40 so that the injection needle 24 is replaceable, therefore the injection needle 24 can be made disposable and sterilization of the injection needle 24 may not be necessary.

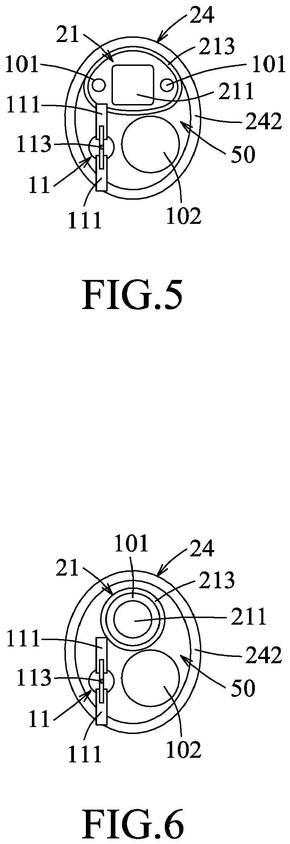

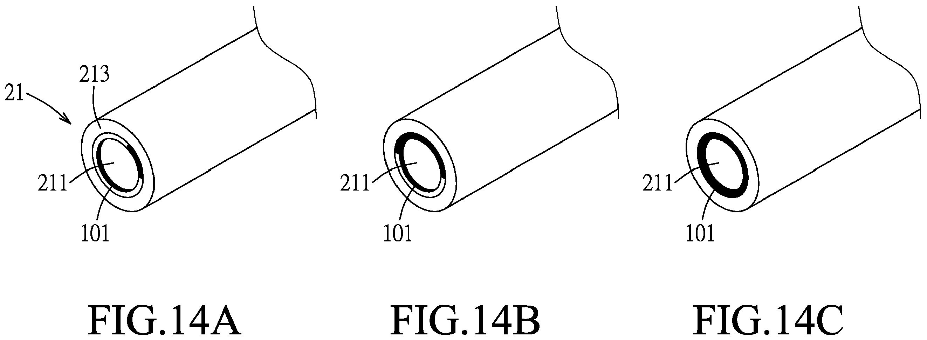

Referring to Figures 1, 2, 5, and 6, the imaging sensing unit 21 includes a tube portion 213 extending from the illumination input end 22 of the base module 40 into the injection needle 24, and an image sensor 211 connected to a distal end of the tube portion 213 and is adjacent to the bevel surface 242 of the injection needle 24. A passage 50 is formed among the connecting rod 12, the tube portion 213 of the image sensing unit 21, and an inner surface of the injection needle 24. In this embodiment, the image sensor 211 may be a traditional optical camera used with a fiberscope, a charge-coupled device (CCD) image sensor, or a complementary metal-oxide semiconductor (CMOS) image sensor, but are not limited to these examples. A processor (not shown) may be disposed in the image sensor 211 to first process the image data before it is sent to the output end 23. Referring to Figure 6, a light source conductor 101 conducts the light source provided from the illumination input end 22. In this embodiment the light source conductor 101 is configured as a plurality of optical fiber strands that are arranged in a circle around the periphery of the image sensor 211. However, the image sensory 211 may be rectangular as shown in Figure 5, and the light source conductor 101 arranged in two bundles on two opposite sides of the image sensor 211. The optical fiber strands of the light source conductor 101 can be arranged to form a complete circle around the image sensor 211 (see Figures 6 and 14C) or only partially lining the periphery of the image sensor 211 as shown in Figures 14A and 14B. Additionally, in other embodiments, the light source conductor 101 may be an LED light source, in this case the light source conductor 101 has an electrical power conductor (electric wire) connecting to the LED that can be arranged on the tip end of the injection needle 24. Furthermore, the outer diameter of the tube portion 213 of the image sensing unit 21 is in a range from 0.8 millimeters to 1.6 millimeters, the diameter (or diagonal length when rectangular) of the image sensor 211 is 0.6 millimeters, and the length of the image sensing unit 21 is in a range from 20 centimeters to 2 meters.

Referring to Figures 1 to 3, the endoscope device 20 further includes a filler member 102, and is convertible between a penetration mode where the filler member 102 extends into the passage 50, and an injection mode where the filler member 102 is removed from the passage. When the injection needle 24 of the endoscope device 20 punctures the patient's skin, the endoscope device 20 is in the penetration mode; this will prevent the patient's tissue from clogging the end hole 243 of the injection needle 24 (the clogging will hinder outlet of the pharmaceutical from the end hole 243), and cause an uneven wound on the patient that is difficult to heal. When the endoscope device 20 is in the injection mode the injection needle 24 may be used to inject the pharmaceutical onto a surgical area, where the pharmaceutical will outlet from the end hole 243 of the injection needle 24. However, in other embodiments of the endoscope assembly 1, a soft tube (not shown) can be extended through the passage 50 and out of the end hole 243 of the injection needle 24, so that surgical irrigation fluid (such as saline) may be used to irrigate the surgical area. Likewise the soft tube may also be used as a surgical drain to drain out the irrigation fluid and other bodily fluids. Additionally a second soft tube (not shown) may be extended through the passage 50 so that the surgical area may be drained as it is irrigated, and according to the requirements of the surgical operation other surgical devices such as monopolar memostatic forceps, bipolar coagulation forceps, harmonic scalpels, electrocauters, and surgical fiber lasers may be extended through the passage 50 as needed instead of the soft tubes.

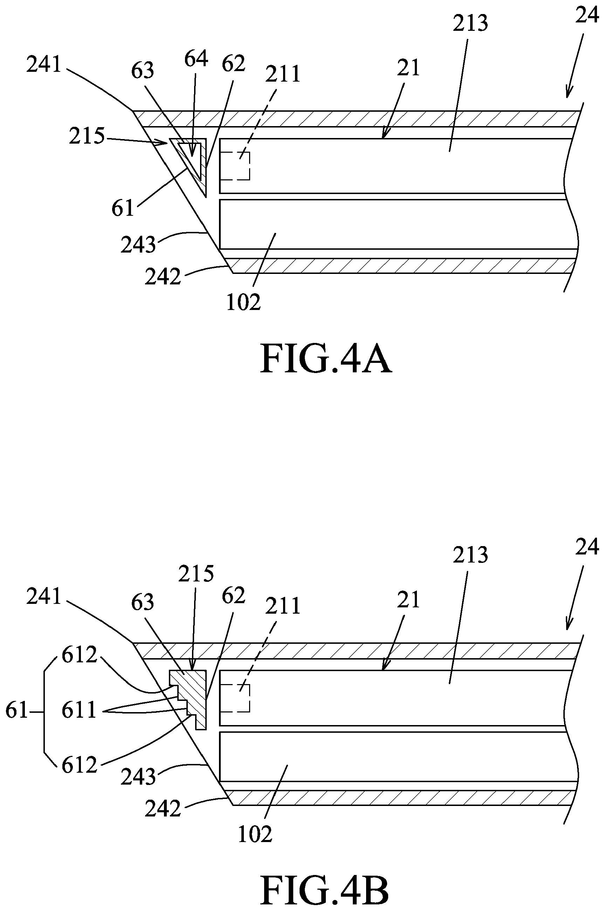

Referring to Figures 1, 4A, and 4B, the optical component 215 is mounted in the injection needle 24 in front of the image sensor 211, under the tip 241 of the injection needle 24 and adjacent to the end hole 243. The optical component 215 is used to help the image sensor 211 resolve the image of the surgical site. The optical component 215 is mounted under the tip 241 so that it is sheathed away from directly impacting the patient's tissue when the injection needle 24 is puncturing a patient's skin, and prevents tearing against the patient's tissue during the puncturing. The wound on the patient will therefore be more even and the healing of the wound will be facilitated compared to a puncture wound with torn tissue. Additionally, this help prevent tissue from building up in front of the image sensor 211 during the puncturing and helps maintain a clear image of the surgical area. The optical component 215 is made from a transparent material (such as glass or plastics), and has a light incident side 61 facing the bevel surface 242, being parallel to the bevel surface 242 of the injection needle 24, and adapted to guide light into the optical component 215. The optical component 215 further has a light emergent side 62 parallel to and facing the end surface of the image sensor 211 and adapted to guide light from the light incident side 61 toward the image sensor 211. In more detail, the optical component 215 is in a beveled frustoconical shape that is beveled at the same incline as the bevel surface 242 (as shown in Figures 4A, 4B). In this embodiment, the light incident side 61 and the light emergent side 62 of the optical component 215 forms an acute angle therebetween.

In this embodiment, the optical component 215 is structurally hollow, and has an inner space 64 between the light incident side 61 and the light emergent side 62. The light emergent side 62 is adapted to guide light that is incident on the light incident side 61 and that is refracted by the inner space 64 to be substantially perpendicular to the end surface of the image sensor 211. Specifically, the hollow structure of the optical component 215 generates minimal interference with light passing through the optical component 215 and refracted by the inner space 64.

Referring to Figure 4B, showing a variation of the embodiment. In this variation, the light incident side 61 is stepped, and has a plurality of spaced-apart first incident surfaces 611 that are parallel to the end surface of the image sensor 211 and that are arranged in a direction parallel to the bevel surface 242 of the injection needle 24. Each of the first incident surfaces 611 has a length that is not larger than one micrometer. The first incident surfaces 611 are micron sized in scale and arranged in a direction parallel to the bevel surface 242, thereby allowing a significant portion of the incident light to pass perpendicularly through the light incident side 61, the light emergent side 62 and onto the image sensor 211, and thereby reducing distortion of the image due to refraction.

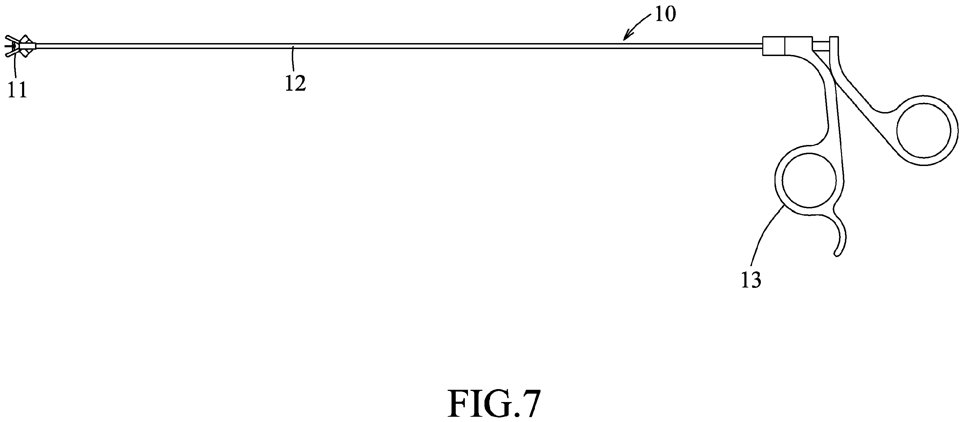

Referring to Figures 1, 3, and 7, the connecting rod 12 has an end connected to the handle 13 of the surgical instrument 10, and extends into the injection needle 24. The forceps device 11 is connected to a distal end of the connecting rod 12 opposite to the handle 13. In this embodiment, the connecting rod 12 is flexible, so that the surgical instrument 10 may be used to extend through the third branch tube 33 into the injection needle 24 via the second instrument port 331, as illustrated in Figure 12.

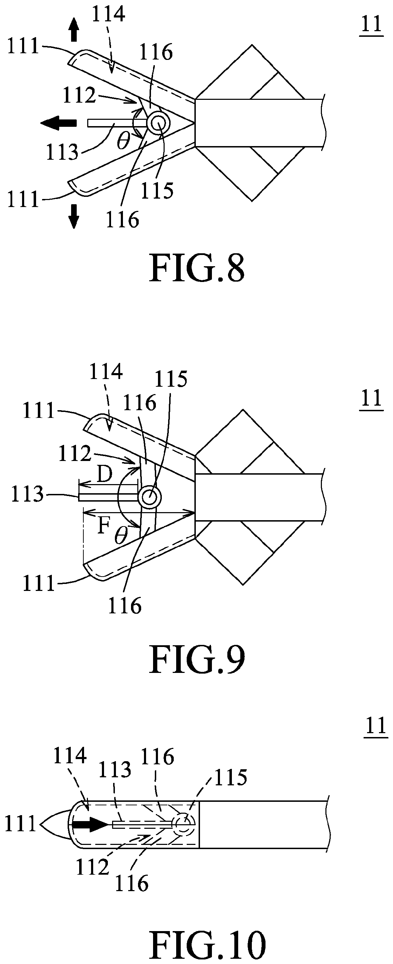

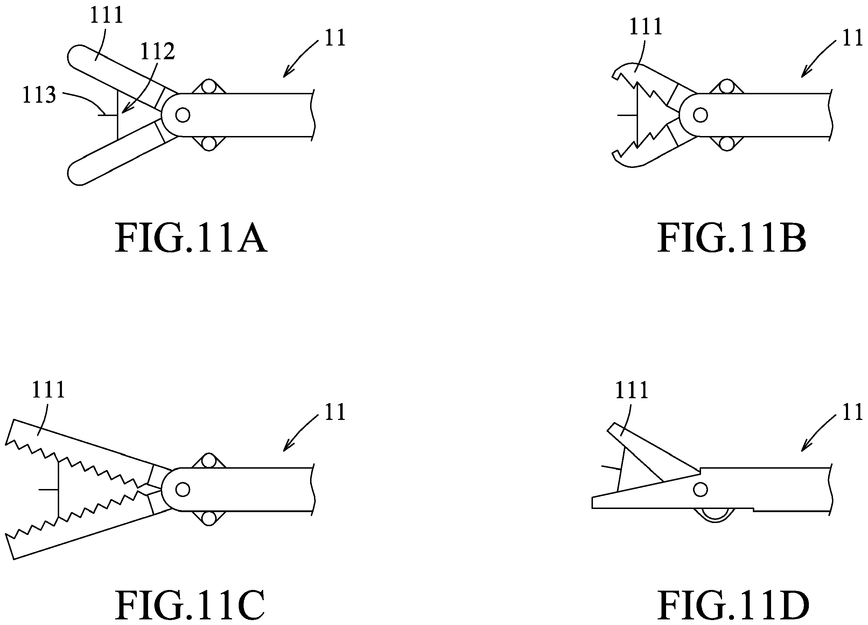

Referring to Figures 8, 9, and 11A to 11D, the forceps device 11 can be used for drug delivery or for holding a medical device. The forceps device 11 has two pincers 111, a hinge set 112 connected between the two pincers 111, and a push rod 113 extending from the hinge set 112 away from the connecting rod 12. The forceps device 11 is convertible between an enclosed state (see Figure 10) where end portions of the two pincers 111 are locked together and closed, and an open state (see Figure 9) where the end portions of the two pincers 111 are separated from each other. Each pincer 111 of the forceps device 11 has an internal space 114, and the internal spaces 114 of the pincers 111 are open toward each other. When the forceps device 11 is in the enclosed state, the internal spaces 114 of the pincers 111 are joined together to accommodate the hinge set 112 and the push rod 113, and drugs and medical devices may also be placed in the internal spaces 114. The pincers 111 can be used to grasp tissue, or retract tissue and organs. The pincers 111 may be in different shapes, for example, in a shape suitable for taking a biopsy sample as shown in Figure 11A, serrated as shown in Figure 11B, serrated and adapted for grasping as in Figure 11C, and scissor like in Figure 11D. The shape of the pincer 111 is not limited to the examples given above and an operator of the endoscope assembly 1 may select the most suitable shape so that the pincers 111 may fulfil the functions of grasping, retracting, incision, resection, excision, etc. Additionally, the tip of the push rod 113 may be rectangular, sharpened or having a serrated edge, or in the shape of a heater shield, however, the tip of the push rod 113 is not limited to the above examples and may take other forms. In some embodiments the forceps device 11 can be configured as a biopsy forceps.

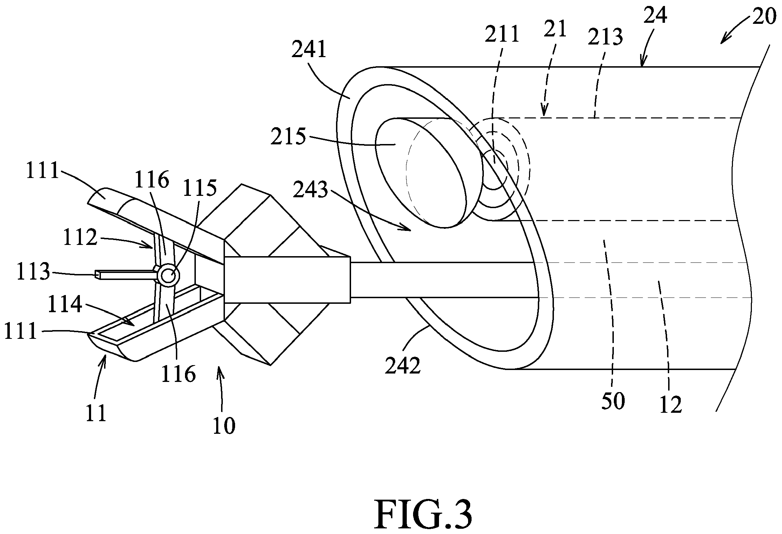

Referring to Figures 3 and 9, variations of the configuration of the forceps device 11 are directed to specific functions. For example, if a ratio of a length (D) of the push rod 113 to a length (F) of each pincer 111 in the axis of the injection needle 24 when the forceps device 11 is in the open state ranges between 0.5 and 1, the push rod 113 is suitable for piercing tissue and taking biopsy samples . The ratio between the length (D) and the length (F) affects the size of the drug that may be placed in the internal spaces 114 of the pincers 111 when the forceps device 11 is in the closed state.

Referring to Figures 3 and 8 to 10, the hinge set 112 of the forceps device 11 has a hinge member 115 and two leg rods 116. Each of the leg rods 116 has opposite end portions that are connected pivotally to the hinge member 115 and a respective one of the pincers 111. Each of the leg rods 116 further has opposite inner and outer longitudinal edges that extends between the opposite end portions and that are respectively proximate to and distal from the connecting rod 12. The push rod 113 and the hinge set 112 are accommodated in the internal spaces 114 of the pincers 111 when the forceps device 11 is in the enclosed state (see Figure 10). Referring to Figure 8, in the conversion of the forceps device 11 from the enclosed state to the open state, the hinge set 112 is driven to operate to thereby move the push rod 113 away from the connecting rod 12. During the conversion of the forceps device 11 from the enclosed state to the open state, a drug contained in the internal spaces 114 may be delivered by the movement of the push rod 113 away from the connecting rod. During the conversion of the forceps device 11 from the open state to the enclosed state, a biopsy sample taken by the push rod 113 may be successfully placed in the internal spaces 114 of the pincers 111. Additionally, when the forceps device 11 is in the open state, an angle (θ) between the outer longitudinal edges of the leg rods 116 is less than 180 degrees so that pivot action of the hinge set 112 may more easily be actuated to convert the forceps device 11 toward the enclosed state. As shown in Figure 9, in this embodiment, the angle (θ) is 150 degrees.

Referring to Figures 2 and 3, before puncturing the patient's tissue, the surgeon converts the endoscope device 20 into the penetration mode (see Figure 2) . Next the forceps device 11 is converted into the enclosed state in order to fit into the injection needle 24, during this stage the surgeon carefully ensures that the forceps device 11 is not protruding out of the bevel surface 242 so as not to compromise the puncturing of the injection needle 24. When a diagnoses of the patient's condition has been made and the surgical area identified, the endoscope assembly 1 can be operated so that the image sensor 211 monitors the surgical site. Next the endoscope device 20 is converted into the injection mode (as shown in Figure 3) to inject the pharmaceutical onto the surgical site. At the same time the handle 13 of the surgical instrument 10 can be operated to assist the surgical operation, and the forceps device 11 can be converted between the enclosed state and the open state for drug delivery and biopsy.

In this embodiment, the output unit 231 is wirelessly connected to the output end 23 of the base module 40 of the endoscope assembly 1, for example, via blue tooth, ZigBee, Wi-Fi, or RF etc. Additionally, the output unit 231 may be a display, a portable electronic device or virtual reality goggles. For example, the surgeon may wear a virtual reality headset as the output unit 231 to view images captured by the image sensor 211, however this is not a limitation on the type of output unit 231 that may be employed.

Referring to Figures 1 and 13, the output unit 231 has a bus 2311 that is connected to the image sensing unit 21 via a data connection, a processor 2312 that is connected to the bus 2311 via a data connection, and a display panel 2313 that is electrically connected to the processor 2312. The bus 2311 is disposed for transmitting an image signal captured by the image sensor 211. The processor 2312 is disposed for receiving the image signal transmitted by the bus 2311 and for processing the image signal. The display panel 2313 is disposed for displaying the image signal processed by the processor 2312.

In summary of the above, by virtue of the forceps device 11 being capable of converting from the enclosed state to the open state to move the push rod 113 away from the connecting rod 12, the operating surgeon can precisely control the surgical instrument 10 to deliver the drug or medical device to the targeted surgical area.

Furthermore, by having the base module 40 removably connected to one of the first and second instrument ports 312, 331, and the surgical instrument 10 extending into the injection needle 24 via the other one of the first and second instrument ports 312, 331, the handle 13 of the surgical instrument 10 is spaced apart from the base module 40. Therefore, there is more room around the handle 13 for the surgeon to maneuver while performing the surgical operation.

Moreover, by virtue of the endoscope assembly 1 having the passage 50, an injection can be directly administered to the surgical area while monitored by the operating surgeon via the image sensor 211 nearby. This will avoid situations where the operating surgeon must constantly re-position the endoscope device 20 so that the surgical area is kept under inspection while performing the injection. Additionally, by virtue of the endoscope assembly 1 including the surgical instrument 10, the surgical instrument 10 can be used during the surgical operation without an additional trocar placement on the patient, thereby reducing the duration of the operation, and preventing unnecessary incisions being made on the patient, which may limit the size and number of incisions and shorten the recovery period. On the other hand, by having the penetration mode the endoscope device 20 can make even needle punctures on the patient, and in the injection mode the passage 50 allows surgical devices to access to the surgical area, thereby avoiding making another incision on the patient or expanding the original incision for better access to the surgical area. The smaller incisions could lead to a quicker recovery for the patient, and require less postoperative care. Therefore, the objects of this disclosure has been satisfied.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. An endoscope assembly (1) including
an endoscope device (20) including
a base module (40) that includes an illumination input end (22),
an injection needle (24) that is tubular, that is connected to said base module (40), and that has a bevel surface (242) formed on a distal end thereof, and
an image sensing unit (21) that includes a tube portion (213) extending from said illumination input end (22) of said base module (40) into said injection needle (24), and an image sensor (211) connected to a distal end of said tube portion (213) and being adjacent to said bevel surface (242) of said injection needle (24);
**characterized in that**:
said endoscope assembly (1) further includes a surgical instrument (10) including
a handle (13),
a connecting rod (12) that has an end connected to said handle (13), and that extends into said injection needle (24), and
a forceps device (11) that is connected to a distal end of said connecting rod (12) opposite to said handle (13), and that has two pincers (111), a hinge set (112) connected between said two pincers (111), and a push rod (113) extending from said hinge set (112) away from said connecting rod (12);
said forceps device (11) is convertible between an enclosed state where end portions of said two pincers (111) are locked together and closed, and an open state where said end portions of said two pincers (111) are separated from each other; and
conversion of said forceps device (11) toward the open state drives said hinge set (112) to operate to thereby move said push rod (113) away from said connecting rod (12).

2. The endoscope assembly as claimed in Claim 1, **characterised in that**:
each pincer (111) of said forceps device (11) has an internal space (114); and
when said forceps device (11) is in the enclosed state, said internal spaces (114) of said pincers (111) are joined together to accommodate said hinge set (112) and said push rod (113).

3. The endoscope assembly as claimed in any one of Claim 1 and 2, **characterised in that**:
said hinge set (112) of said forceps device (11) has a hinge member (115) and two leg rods (116), each of said leg rods (116) having opposite end portions that are connected pivotally to said hinge member (115) and a respective one of said pincers (111), each of said leg rods (116) further having opposite inner and outer longitudinal edges that extends between said opposite end portions and that are respectively proximate to and distal from said connecting rod (12); and
when said forceps device (11) is in the open state, an angle (θ) between said outer longitudinal edges of said leg rods (116) is less than 180 degrees.

4. The endoscope assembly as claimed in any one of Claims 1 to 3, **characterised in that**:
said endoscope device (20) further includes an adaptor (30) having a connection port (311), a first instrument port (312), an injection port (321), and a second instrument port (331) that are in fluid communication with each other;
said connection port (311) is connected to said injection needle (24);
said injection port (321) being adapted for inlet of a pharmaceutical therethrough into said injection needle (24); and
said base module (40) is removably connected to one of said first instrument port (312) and said second instrument port (331), and said surgical instrument (10) extends into said injection needle (24) via the other one of said first instrument port (312) and said second instrument port (331), so that said handle (13) of said surgical instrument (10) is spaced apart from said base module (40).

5. The endoscope assembly as claimed in Claim 4, **characterised in that**:
said adaptor (30) includes a first branch tube (31) that is staight and that has two opposite ends being respectively formed with said first instrument port (312) and said connection port (311); and
said surgical instrument (10) extends through said first branch tube (31) via said first instrument port (312) into said injection needle (24).

6. The endoscope assembly as claimed in Claim 4, **characterised in that**:
said adaptor (30) includes
a first branch tube (31) that is staight and that has two opposite ends being respectively formed with said first instrument port (312) and said connection port (311),
a second branch tube (32) that is connected to said first branch tube (31) and that has said injection port (321), and
a third branch tube (33) that obliquely extends from said first branch tube (31) away from said connection port (311), and that has said second instrument port (331);
said third branch tube (33) and said first branch tube (31) form an acute angle therebetween; and
said connecting rod (12) of said surgical instrument (10) is flexible, said surgical instrument (10) extending through said third branch tube (33) via said second instrument port (331) into said injection needle (24) .

7. The endoscope assembly as claimed in any one of Claims 1 to 6, **characterised in that**:
a passage (50) is formed among said connecting rod (12), said tube portion (213) of said image sensing unit (21), and an inner surface of said injection needle (24);
said endoscope device (20) further includes a filler member (102); and
said endoscope device (20) is convertible between a penetration mode where said filler member (102) extends into said passage (50), and an injection mode where said filler member (102) is removed from said passage (50).

8. The endoscope assembly as claimed in any one of Claims 1 to 7, **characterised in that** said injection needle (24) is removably connected to said base module (40) so that said injection needle (24) is replaceable.

9. The endoscope assembly as claimed in any one of Claims 1 to 8, **characterised in that**:
said image sensor (211) has an end surface that is perpendicular to an axis of said injection needle (24);
said bevel surface (242) of said injection needle (24) is inclined relative to said end surface of said image sensor (211);
said endoscope device (20) further includes an optical component (215) that is made from a transparent material, that is mounted in said injection needle (24) in front of said image sensor (211), and that has
a light incident side (61) mounted adjacent to and being parallel to said bevel surface (242) of said injection needle (24), and adapted to guide light into said optical component (215), and
a light emergent side (62) parallel to and facing said end surface of said image sensor (211), and adapted to guide light from said light incident side (61) toward said image sensor (211); and
said light incident side (61) and said light emergent side (62) forms an acute angle therebetween.

10. The endoscope assembly as claimed in Claim 9, **characterised in that**:
said optical component (215) is structurally hollow, and has an inner space (64) between said light incident side (61) and said light emergent side (62); and
said light emergent side (62) is adapted to guide light that is incident on said light incident side (61) and that is refracted by said inner space (64) to be substantially perpendicular to said end surface of said image sensor (211).

11. The endoscope assembly as claimed in any one of Claims 9 and 10, **characterised in that** said light incident side (61) is stepped, and has a plurality of spaced-apart first incident surfaces (611) that are parallel to said end surface of said image sensor (211) and that are arranged in a direction parallel to said bevel surface (242) of said injection needle (24), each of said first incident surfaces (611) having a length that is not larger than one micrometer.

12. The endoscope assembly as claimed in any one of Claims 1 to 11, **characterised in that** said base module (40) further includes a relay mechanism (25) that is configured as one of an optical fiber light concentrator, a multimedia interface, and an LED light power source.

13. The endoscope assembly as claimed in any one of Claims 1 to 12, **characterised in that** said surgical instrument (10) is configured as a biopsy forceps.

14. An endoscope system **characterised by**:
said endoscope assembly as claimed in any one of Claims 1 to 13, wherein said base module (40) further has an output end (23); and
an output unit (231) connected to said output end (23) of said base module (40) of said endoscope assembly, and having a bus (2311) that is connected to said image sensing unit (21) via a data connection, a processor (2312) that is connected to said bus (2311) via a data connection, and a display panel (2313) that is electrically connected to said processor (2312);
said bus (2311) being disposed for transmitting an image signal captured by said image sensor (211), said processor (2312) being disposed for receiving the image signal transmitted by said bus (2311) and for processing the image signal, said display panel (2313) being disposed for displaying the image signal processed by said processor (2312).

15. The endoscope system as claimed in Claim 14, **characterised in that** said output unit (231) is connected to said output end (23) of said base module (40) of said endoscope assembly via a wireless connection.
